Europäisches Patentamt

European Patent Office

Office européen des brevets

(19)

(11) Publication number: **0 168 127**
**A2**

(12) **EUROPEAN PATENT APPLICATION**

(21) Application number: **85302225.9**

(22) Date of filing: **29.03.85**

(51) Int. Cl.⁴: **C 12 P 19/14**
**C 12 M 1/40**

(30) Priority: **16.04.84 US 601062**

(43) Date of publication of application:
**15.01.86 Bulletin 86/3**

(84) Designated Contracting States:
**BE DE FR GB IT NL SE**

(71) Applicant: **AMERACE CORPORATION**
**555 Fifth Avenue**
**New York, NY 10017(US)**

(72) Inventor: **Goldberg, Bruce S.**
**90 Parson Road Clifton**
**Bergen New Jersey(US)**

(74) Representative: **Howick, Nicholas Keith et al,**
**Carpmaels & Ransford 43 Bloomsbury Square**
**London WC1A 2RA(GB)**

(54) **A process and apparatus to hydrolyze lactose to glucose and galactose.**

(57) A method and apparatus for the continuous hydrolyzation of lactose into glucose and galactose employing a flow-through reactor upon which has been immobilized a lactase system. A continuous stream of a lactose solution is introduced, either from lactose powder crystalized from a whey stream or directly from one or more ultra-filtration devices which remove the whey protein concentrate from a whey stream and give a proper lactose solution.

Various devices are provided to adjust the temperature, pH and size of solids in suspension during the conversion process. Means are provided to prevent the growth of bacteria in the lactose solution and to minimize suspended matter in the solution. The flow-through reactors may take the form of stacked microporous sheets or may have an overall spiral jelly-roll configuration.

EP 0 168 127 A2

A PROCESS AND APPARATUS TO HYDROLYZE
LACTOSE TO GLUCOSE AND GALACTOSE

BRUCE S. GOLDBERG

## BACKGROUND OF THE INVENTION

### Field of the Invention

The invention is directed to the field of hydrolyzing common basic materials into higher order more valuable materials employing immobilized proteinacious preparations bonded to a flow-through reactor. More particularly, a lactose solution is hydrolyzed by a lactase system immobilized on a flow-through reactor to hydrolyze substantially all of the lactose to glucose and galactose.

### Description of the Prior Art

At present lactose is hydrolyzed to glucose and galactose using lactase only in a batch approach. All the required ingredients, including the lactose solution and the lactase enzyme are placed in a large vat which must be heated to prevent the growth of bacteria therein and the contents of the vat has its pH adjusted to the desired level and sufficient time is alloted for the conversion of the lactose to glucose and galactose. The solution is now filtered to remove the non-converted lactose and the lactase enzyme and any other impurities. The enzyme

-1-

is at this point considered an impurity since it may 0168127
affect the taste, odor, color or appearance of the
finished product and is removed and discarded.
Since the vat size has certain physical limitations
and the enzyme is used but once the cost of the hydrolyzation is high and the process is inefficient.

SUMMARY OF THE INVENTION

The process and apparatus to hydrolyze lactose to
glucose and galactose disclosed herein overcomes the
difficulties noted above with respect to prior art
batch processes by providing a process and apparatus
which operate on a continuous basis, to hydrolyze a
continuous stream of a lactose solution employing an
enzyme system which is immobilized on a flow-through
reactor to provide glucose and galactose from substantially all of the lactose solution provided.
The immobilization of the lactase system on the
flow-through reactor permits its use for thousands
of hours rather than the short, single batch term
for which they are now used.

A stream of a lactose solution, which may be
approximately 5% lactose, obtained from dissolving
lactose crystals in water or employing the whey
stream from one or more ultra-filtration devices in
series, is adjusted as to its pH in a feed tank.

At the same time the solution is treated to prevent the growth of bacteria by heating it to a predetermined temperature for a predetermined period, this process being better known as pasturization, or the solution is recirculated through an ultra-violet radiation unit. The pasturization step will require that the solution be cooled to the proper temperature before introduction into the flow-through reactor. The reactor may take the form of a stack of microporous sheets with the solution introduced perpendicular to the major surface of the sheets or it may take the form of a spiral jelly-roll configuration with the solution introduced into its porous core or its open free end. The solution moves generally between the reactor convolutions but may also pass through the microporous sheets which define the reactor convolutions.

Various filters, detection devices, recorders, etc. are employed to maximize the overall efficiency of the conversion process. It is an object of this invention to provide a method and apparatus for the continuous conversion of lactose to glucose and galactose using a flow-through reactor.

It is an object of this invention to provide a method and apparatus for the continuous conversion of lactose to glucose and galactose where said lactose is provided as a solution from the dissolving of crystals of lactose in water.

It is another object of this invention to provide a method and apparatus for the continuous conversion of lactose to glucose and galactose where said lactose is provided as a solution from whey permeate.

It is still another object of this invention to provide a method and apparatus for the continuous conversion of lactose to glucose and galactose where said lactose is provided as a solution from whey permeate discharged from an ultra-filtration system.

It is yet another object of this invention to provide a method and apparatus for the continuous conversion of lactose to glucose and galactose where said lactose is provided as a solution from whey permeate discharged from an ultra-filtration system comprising at least two ultra-filtration units connected in series.

It is still a further object of this invention to provide a method and apparatus for the continuous converion of lactose to glucose and galactose where said lactose is provided as a solution from whey permeate discharged  from an ultra-filtration system comprising at least  two ultra-filtration units in series with a clarity determining device that

-4-

recirculates the output whey permeate back to one of the ultra-filtration units if the clarity is too low indicating that the whey permeate contains too much suspended solid matter.

It is another object of this invention to provide a method and apparatus for the continuous conversion of lactose to glucose and galactose and where the lactose is in a solution continuously recirculated through a source of ultra-violet radiation prior to processing to prevent the growth of bacteria in said solution.

Other objects and features of the invention will be pointed out in the following description and claims and illustrated in the accompanying drawings, which disclose, by way of example, the principles of the invention, and the best modes which have been contemplated for carrying them out.

## BRIEF DESCRIPTION OF THE DRAWINGS

In the drawings in which similar elements are given similar reference characters:

FIG. 1 is a schematic representation of a first form of apparatus for the continuous hydrolyzation of lactose into glucose and galactose in accordance with the concepts of the invention.

FIG. 2 is a schematic representation of a further embodiment of an apparatus for the continuous hydrolyzation of lactose into glucose and galactose in accordance with the concepts of the invention.

FIG. 3 is a schematic representation of a further embodiment of an apparatus for the continuous hydrolyzation of lactose into glucose and galactose in accordance with the concepts of the invention.

FIG. 4 is a schematic representation of one form of bacteria growth prevention system which can be employed with the apparatus of FIG. 3.

FIG. 5 is a schematic representation of a second form of bacteria growth prevention system which can be employed with the apparatus of FIG. 3.

FIG. 6 is a detailed schematic representation a yet another embodiment of an apparatus for the continuous hydrolyzation of lactose into glucose and galactose and showing the various devices employed to clean the major components of such apparatus.

FIG. 7 is a schematic representation of a subsystem used to determine the amount of solid material suspended in the lactose solution.

-6-

## DESCRIPTION OF THE PREFERRED
## EMBODIMENTS

The basic concept of the present invention is to provide a method and apparatus for the continuous hydrolyzation of lactose in a lactose solution into glucose and galactose by passing such lactose solution through a flow-through reactor which has immobilized thereon a proteinacous substance such as the enzyme lactase. The enzyme, because it is immobilized on a suitable porous or specially configured support, does not become a part of the glucose and galactose and can be used a great number of times making the conversion operation far less expensive. Also because the system is a continuous one, the equipment required is greatly reduced. For example no large vat and heating system for such vat is required.

In order that the reactor be used properly it is necessary to adjust the pH, the temperature and the transit time of the solution through the flow-through reactor. Excess solids must be removed from the converted solution based upon the reactor system employed.

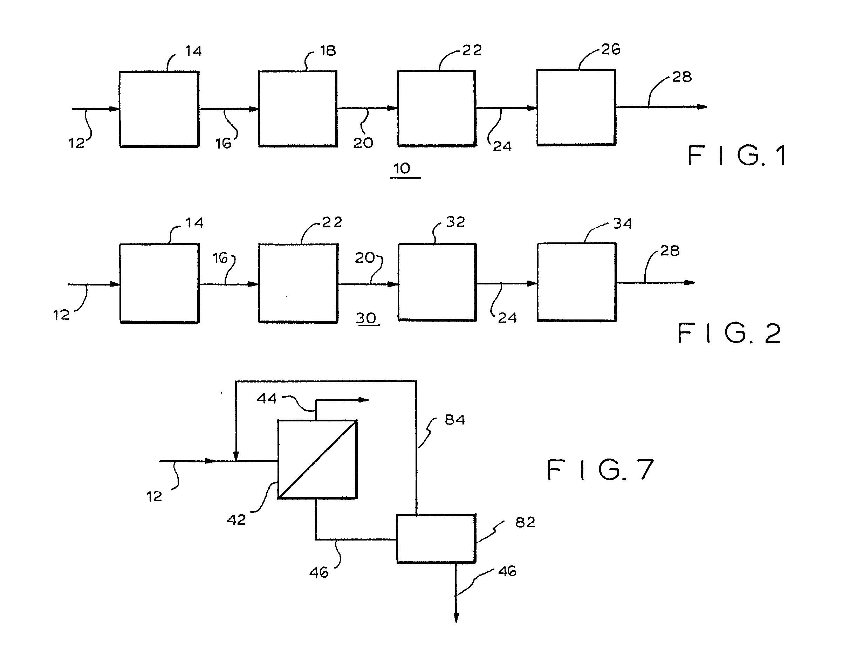

Turning now to FIG. 1 there is shown a first form of apparatus 10 for the continuous

-7-

hydrolyzation of lactose into glucose and galactose.
Lactose crystals obtained, for example, from the
crystallization of lactose found in milk whey is
dissolved in water to give a 5% lactose solution
and supplied via input line 12 to a feed tank 14
used to maintain a constant flow of lactose solution
through the apparatus 10. Although the solution is a
5% solution, the effective lactose concentration
can be in the range of          to          or
higher. Due to the possible presence of unwanted
materials such as fat globules, salts, trace amounts
of protein or other milk solids which can block the
pores of the flow-through reactor, as hereinafter
set forth, the lactose solution is passed via line
16 to a filter 18. Filter 18 can be made up of a
single unit to pass only particles below a given
size or can be constructed of a series of graduated
filter elements so that particles below a given can
be passed through the last filter element.

The filtered lactose solution is then passed
via line 20 into a tank 22 where the lactose solution is heated to the desired temperature and the
pH is adjusted. The incoming lactose solution is
generally slightly acidic having a pH of approximately 6.5. The lactase used in the reactor is an
acid lactase and accordingly the lactose solution must be made more acidic to a pH of 4.5. This is accomplished by the addition to the lactose solution in tank 22 of sulphuric acid, hydrocloric acid or acetic acid. For the particular lactase enzyme system chosen the temperature of the lactose solution must be raised to a temperature of 30° to 40° C from room temperature of approximately 21° C. This may be accomplished, for example, by the use of an immersed heating coil (not shown) in tank 22. The lactose solution, now at the desired temperature and pH is conducted via line 24 into the reactor 26.

Alternatively, the positions of the filter 18 and the tank 22 can be reversed. This may be desireable in situations where the acid used to adjust the pH has not been filtered and any particles present must be removed before the solution is passed to the reactor 26 or in case the pasturization of the solution causes coagulation and the precipitants must also be removed prior to introduction into reactor 26.

Reactor 26 is made up of one or more reactor modules (not shown), each of which comprises a plurality of stacked microporous sheets having a plurality of pores extending from their respective surfaces. These pores are not continuous, but rather form a random tortuous pathway. One type of microporous sheet is shown and described in United States Letters Patent No 4,102,746 issued 25 July 1978 in the name of Bruce S. Goldberg and assigned to the assignee of the instant invention. In addition other pastic and rubber sheets can be used. Proteinaceous preparation, for example exzymes, can be immobilized upon the silica frag ments on the surface of the sheeting and in the pores of the sheeting employing the techniques described in the 4,102,746 patent and in United States Letters Patent No 4,169,014 issued 25 September 1979 in the name of Bruce S. Goldberg and also assigned to the assignee of the instant invention. Other suitable techniques are also known in the prior art.

In the instant case reactor 26 has an acidic lactase enzyme system immobilized on the microporous sheets of the reactor 26. By controlling the pressure and volume of the lactose solution applied to the reactor 26, and by employing a suitable number of micorporous sheets in the reactor modules as well as the configuration of the internal duct work of the reactor 26 (not show), the time required to pass through reactor 26, called the transit time, can be controlled to give a high rate of conversion of the lactose to glucose and galactose. It has been found that employing a 5% lactose solution at $30^{\circ}$ C, with a pH of 4.5 and a transit time of 2 minutes through reactor 26, there is a 90% conversion of the lactose to glucose and galactose. These components as well as the unconverted lactose solution is fed via output line 28 to suitable

-10-

storage or immediate use as a sweetener or food ingredient. If desired the non-converted lactose may be removed before the glucose and galactose are employed.

To use the reactor 26 as described above is is necessary to carefully filter the lactose solution to remove any fat globules, coagulants, cheese or milk solids which are present and which singlely or in combination can block the pores of the reactor and prevent the flow through of the lactose solution. This condition is sometimes referred to in the art as "blinding" the reactor or filter. To permit the use of unfiltered feedstocks and to prevent the accidental blinding of the reactor, a spiral reactor may be employed instead. This reactor, also made up of microporous sheets, is formed into a jelly-roll spiral configuration with each convolution separated from adjacent convolutions by spacing means which permit the solution to flow between the convolutions and in contact with the sheet surfaces upon which the enzyme is immobilized. A porous core and an open free end permit the lactose solution to be placed in the reactor and the reaction products removed. Such a jelly-roll spiral reactor is disclosed in a copending application for a United States Letter

-11-

Patent Serial No        filed in the name of Bruce S. Goldberg and Richard Y. Chen entitled "Spiral Designed Reactor" and assigned to the assignee of the instant invention.

One form of apparatus 30 using a jelly-roll spiral configured reactor 32 is shown in FIG 2. The lactose solution is supplied via input line 12 to feed tank 14. The output of feed tank 14 passes line 16 to tank 22 where the lactose solution is heated to the desired temperature and the pH is adjusted. The output of tank 22 is feed via line 20 to the spiral reactor 32. Since the output of reactor 32 may contain undesired solids and fat these may be removed by means of filter 34 arranged to remove the undesired materials passed to filter 34 via line 24. The filtered glucose and galactose and remaining unconverted lactose solution is available on output line 28 for whatever is its intended use.

In the making of cheese an enzyme called rennet is added to the whole milk to curdle it. The curd is the cheese and the remainder is the whey. Whey is about 93 to 94% water and 6 to 7% solids consisting of protein, salts such as sodium chloride, potassium phosphate and lactose. On a weight basis

-12-

for each 10 pounds of whole milk, there is 1 pound of cheese and 9 pounds of whey. In the previous examples, the lactose was separated out from the whey and crystallized and the lactose solution was produced by dissolving the lactose crystals in water. The lactose solution can also be obtained directly from the whey by the use of a membrane type separation device or ultra-filtration system of the type commercially available from DEC International Inc. of Fond Du Lac, Wisconsin and having a 20,000 to 40,000 molecular weight cut-off. The ultra-filtration system receives the whey and produces at one output whey protein concentrate or retentate and at the second output permeate which is comprised of 95% water, 1/2% salts, 4 1/2% lactose and trace amounts of protein. The dried protein powder can be used as a food enricher when suitably dried. The presence of residual protein in the whey permeate can be detected and the permeate run through a second ultra-filtration system called a polisher which is like the one described above except that the molecular weight cut-off is 20,000. Alternatively, upon the detection of more than an insignificant amount of residual protein, the permeate can be recirculated through the first ultra-filtration unit again.

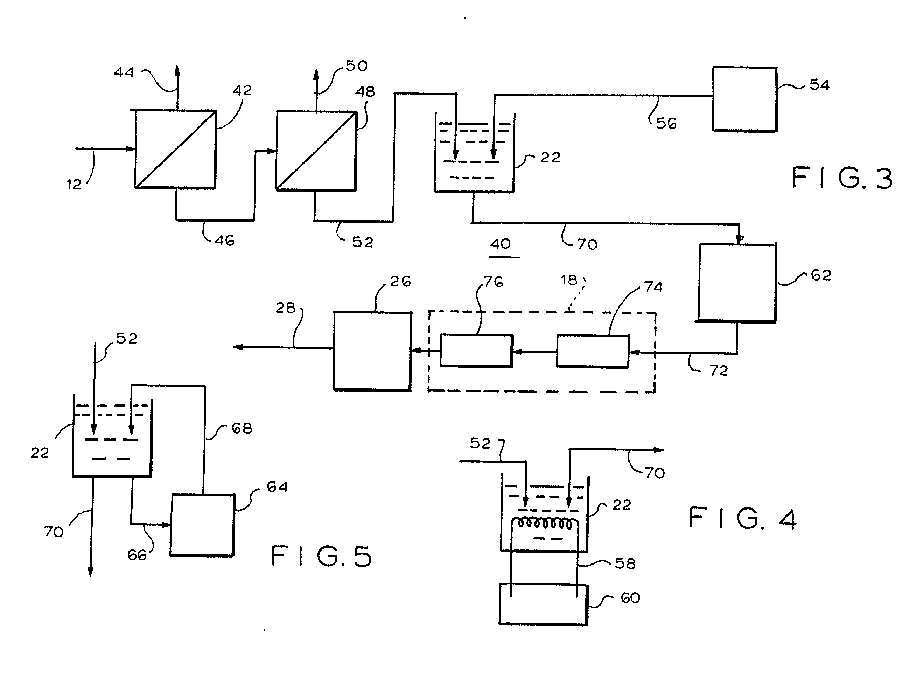

FIG. 3 shows an apparatus 40 for the continuous hydrolyzation of lactose into glucose and galactose and employing whey as the starting material. Whey from the cheese making vats is feed via input line 12 into ultra-filtration system 42. At output line 44 the whey protein concentrate or retentate is removed while the whey permeate is available at output line 46. The permeate is applied to the ultra-filtration polisher 48 to remove any residual protein which passed through ultra-filtration system 42. Protein removed by polisher 48 is available on line 50 while the permeate is available on line 52. The permeate is placed in vat 22 to have its pH adjusted. The pH of the incoming permeate is determined and sufficient acid to lower the pH of the permeate in vat 22 is added by the device 54 connected to vat 22 by line 56. Devices to perform this function are sold by Horizon Incorporated.

In that the lactose solution in vat 22 is an ideal growth medium for bacteria, something must be done to prevent such growth. Two procedures are available to destroy the bacteria present in vat 22 and prevent their growth. A first, akin to pasturization, amounts to raising the temperature of the lactose solution in vat 22 to approximately 160° F

-14-

(71° C) for a prescribed period and the second requires the continuous recirculation of the lactose solution through a source of ultra-violet radiation to irradiate such solution. The heating technique is shown in FIG 4 where a heating coil 58 coupled to a thermostatic control 60 is immersed in the lactose solution in vat 22. Since the temperature of the lactose solution is raised to approximately 71° C it is necessary that the solution in line 70 be cooled in cooler 62 before it is applied to reactor 26.

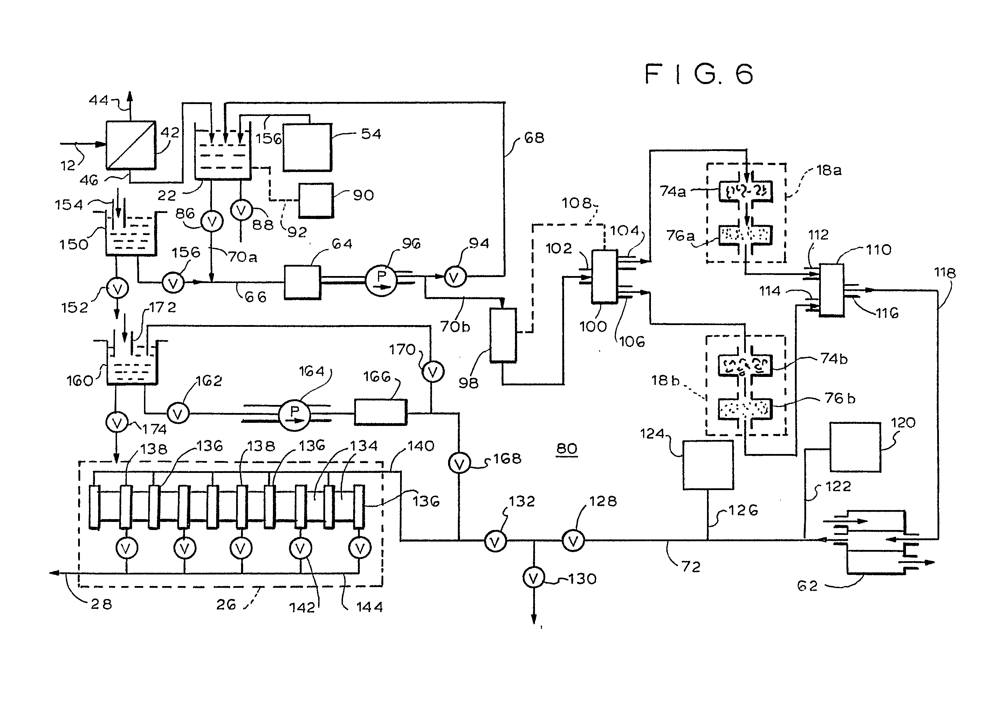

Alternately, a source of ultra-violet radiation 64, as shown in FIG 5 can be used to continually irradiate the lactose solution as it is recirculated from vat 22, via line 66 to the ultra-violet radiation source 64 and back to vat 22 via line 68.

This constant recirculation kills any bacteria present. Since the irradiation is carried out at room temperature, approximately 68° F or 21° C it is necessary to warm the solution temperature to the desired 30° to 40° C range. This can be done by the use of a heater such as 58, 60 in FIG 4 in vat 22 (not shown). Also since the solution will be raised to the correct temperature in vat 22 no cooler such as 62 needs to be employed. The cooler 62 may

-15-

be made using coils (not shown) in unit 62 through which refrigerated water is passed.

The lactose solution of proper pH and temperature is now conducted via line 72 to the filter unit 18 which is made up of a 2 micron filter cartridge of the type made by Cox Manufacturing Company followed by a 0.45 micron filter cartridge of the type made by Gelman Filter Company. After filtration in filter 18, the 5% lactose solution adjusted to the proper pH 4.5, heated to $30^{o}$C, and having all solids above a predetermined size removed is applied to reactor 26. As with the apparatus 10 and 20, the apparatus 40 will achieve a 90% conversion rate with a two minute transit time in the reactor 26. The glucose and galactose and non-converted lactose are available on output line 28.

Turning now to FIG 6 a more detailed apparatus 80 is shown along with some of the housekeeping equipment required to keep all components of the system clean especially necessary when the end products are intended for human consumption. Only a single ultra-filtration system 42 is shown but it should be understood that if required or desired a polisher can be added. Alternatively a feed back system can be employed to cause the permeate to be returned to the ultra-filtration system input if it contains two much suspended matter such as protein. FIG 7 shows how this can be done using a turbidity meter 82. The output line 46 from ultra-filtration system 42 is fed to turbidity meter 82. Normally this permeate should be clear and sparkling and have a turbidity reading of much less than 3 national turbidity units (NTU). If this is the case the permeate is passed to the continuation of output line 46 to vat 22. If the turbidity is above 3 NTU then the solution is gated to line 84 which returns it to input line 12 for a second pass through the ultra-filtration system 42. An alarm system can be connected to turbidity meter 82 to advise of successive failures to attain the desired turbidity level indicating trouble with the ultra-filtration system 42 (ie a ruptured membrane or the like).

Returning to FIG 6, the permeate from ultra-filtration system 42 is placed in vat 22, which also receives acid from pH control 54 via line 56, and the recirculated lactose solution from the ultra-violet radiation unit 64 via recirculation line 68. A valve 86 controls the flow of the lactose solution from vat 22 via lines 79 and 66 to the ultra-violet radiation unit 64. A valve 88 is provided to permit the draining of the solution in vat 22. To prevent settling in the vat 22 a mechanical agitator 90 is coupled by linkage 92 to vat 22. Valve 94 controls the flow of the solution in the recirculation line 68 and pump 96 provides the necessary head for movement of the solution.

Valve 94 is open approximately half of the time so that half of the solution in the recirculation path can be directed to the filtration systems 18a and 18b. The solution leaving the recirculation path passes through flow meter 98 to the distribution valve 100 input 102 and from first output 104 to the first filter unit 18a made up of course filter 74a (2 micron) and fine filter 766 (0.45 micron). The pump 96 is arranged to provide a constant rate of flow-through flow meter 98 to the first unit 18a. When the filter unit 18a is clean and the solution flows easily through filter unit 18a the power required by the pump 96 is constant. However, as fat globules, coagulants, cheese solids and other materials deposit on the filter materials of filter unit 18a and blind them the flow-through flow meter 98 slows and the pump 96 is required to apply more force to the solution, drawing more current and trying to maintain a constant flow-through flow meter 98. When the rate of flow-through flow meter 98 falls below a predetermined value, flow meter 98 operates distribution valve 100 via mechanical linkage 108 to switch its output to its second output 106 which in turn is connected the second filter unit 186 made up of course filter 74b (2 micron) and fine filter 76b (0.45 micron). Flow meters of the type described are available from Brooks Flow Meter Co. When second filter unit 18b blinds the entire apparatus 80 will be shut down for cleaning.

The outputs from first filter unit 18a and second filter unit 18b are applied to inputs 112 and 114, respectively of valve 110 which in turn applies its output on 116 to line 118 leading to the cooling unit or heat exchanger 62. Heat exchanger 62 corrects the temperature of the lactose solution applied to line 72 to that required for proper operation of the reactor 26. A temperature recorder 120 is connected via line 122 to record the temperature of the solution flowing in line 72. Similarly, a pH recorder 124 is coupled via line 126 to record the pH of the solution flowing in line 72. Both recorders 120 and 124 can be equipped with alarms to signal that the solution flowing in line 72 is outside of the desired temperature and pH range.

-19-

Valves 128 and 132 control the flow of the lactose solution in line 72 to the reactor 26. Valve 130 is provided to permit the line 72 to be drained or directed elsewhere. The reactor 26 is made up of a plurality of reactor modules 134 separated from adjacent modules by the plates 136 and 138. The plates 136 are each coupled to the input header 140 to apply the lactose solution to the two modules 134 adjacent thereto. Alternating with the plates 136 are plates 138 each coupled through a valve 142 to the output header 144 and in turn to the output line 28. The plates 138 prevent the solution from going beyond the modules adjacent thereto so that if these modules become contaminated in any way the contaminents will not reach any additional modules. Also this arrangement insures a more even distribution of the solution throughout the reactor 26. Similarly the plates 138 only cooperate with the modules 134 directly adjacent further facilitating substantially even flow throughout the reactor. Valves 142 can be used to limit or cut off flow from the plates 138 to the output header 144 if desired. Normally the valves 142 are fully open. Although only nine reactors 134 are shown this number can be increased or decreased as desired.

Since the reaction products can be used for 0168127
human consumption and are based upon dairy components the overall apparatus must be cleaned
on a regular basis. Also it should be noted
that most of the tanks, piping, valves and similar elements are constructed of stainless steel.
A clean-in-place solution is stored in tank 150
which can be drained through valve 152 if required. The cleaning solution can only be used
for a single pass through the apparatus 80.
Thus more solution can be entered into tank 150
via inlet pipe 154. The cleaning solution in tank
150 is permitted to enter the apparatus 80 by
opening the valve 156 which connects to feed line
66. To prevent the backflow of the cleaning
solution into vat 22 valve 86 is closed. It
should be noted that the valves used in apparatus
80 may be manually operated or electrically
operated at the valve itself or remote therefrom.
The cleaning solution passes through the ultra-
violet radiation system (not operative at such
times), pump 96 and valve 94 into vat 22. Valve
94 is then closed and the cleaning solution is
directed through flow meter 98 to valve 100 which
alternately applies the solution to the first
filter unit 18a and then the second filter unit 18b. Valve 110 applies the cleaning solution to heat exchanger 62 and through valves 128 and 130 to the drain. Valve 132 is closed at this time to ensure that the solution does not reach reactor 26. A buffer solution is now applied to the reactor 26 from tank 160 via valve 162, pump 164, flow meter 166 and valve 168 to input header 140. The buffer solution can also be recirculated back to tank 160 prior to use on reactor 26 by opening valve 170. New buffer solution can be added to tank 160 via inlet pipe 172 and the tank 160 can be drained via valve 174.

While there have been shown and described and pointed out the fundamental novel features of the invention as applied to the preferred embodiments, it will be understood that the various omissions and substitutions and changes of the form and details of the devices illustrated and in their operation may be made by those skilled in the art, without departing from the spirit of the invention.

Further aspects of the present invention are as follows:-

A.    Apparatus as defined in claim 8, further comprising first filter means coupled to said second means to remove all particles larger than a predetermined size.

B.    Apparatus as defined in claim 1, further comprising: temperature correction means coupled between said temperature and pH control means and said flow-through reactor means to adjust the temperature of said solution prior to its entry into said reactor.

C.    Apparatus as defined in claim B wherein said temperature control means is a heater and said temperature correction means is a cooler.

D.    Apparatus as defined in claim 1 wherein said solution contains approximately 1 to 30% lactose in water.

E.    Apparatus as defined in claim 9 further comprising: first filter means coupled between said first output line of said feed tank and said flow-through reactor to remove any particles that could accumulate in said flow-through reactor means and impede its operation.

F.    Apparatus as defined in claim 9 further comprising: first filter means for removing particles larger than a first predetermined size coupled to first output line of said feed tank; second filter means for removing particles larger than a second predetermined size, said second predetermined size being smaller than said first predetermined size, said second filter means coupled to said first filter means and said flow through reactor, said first filter means and said second filter means removing any particles that could accumulate in said flow-through reactor and impede its operation.

G.    Apparatus as defined in claims 1 or 2 or 3 or 4 or 5 or 6 or 7 or 8 or 9 or A or B or C or D or E or F wherein said feed means is ultra filtration means receiving a continuous stream of whey permeate and providing a continuous supply of a solution containing approximately 5% lactose in water.

H.    Apparatus as defined in claims 1 or 2 or 3 or 4 or 5 or 6 or 7 or 8 or 9 or A or B or C or D or E or F wherein said feed means comprises: first ultra-filtration means receiving a continuous stream of whey permeate and separating same into a retentate of whey protein concentrate and a continuous supply of a solution containing approximately 5% lactose in water; a second ultra-filtration means coupled to said first ultra-filtration means for removing from said solution any residual protein not removed by said first ultra-filtration means.

J.    Apparatus as defined in claim G, further comprising: turbidity meter means coupled to the output of said ultra-filtration means and to the input thereof, said turbidity meter means passing solution having a turbidity below a predetermined level and returning solution having a turbditiy above such predetermined level to the input of said ultra-filtration means.

K.    Apparatus as defined in claim H, further comprising: turbidity meter means coupled to the output of said second ultra-filtration means and to the input thereof, said turbidity meter means passing solution having a turbidity below a predetermined level and returning solution having a turbidity above such predetermined level to the input of said second ultra-filtration means.

L.    Apparatus as defined in claims 9 or E, further comprising; agitation means coupled to said feed tank to agitate same and prevent any separation of the materials in said feed tank.

M.    Apparatus as defined in claim E wherein said first filter means comprises; first and second coarse filters and first and second fine filters, said first coarse filter and first fine filters being coupled together to form a first filter branch, said second coarse and said second fine filters being coupled together to form a second filter branch; said apparatus further comprising; an output header connected to the free ends of said first and second fine filters; a selectively operable input header coupled to the free ends of said coarse filters to selectively allow the flow of the solution through said first or second filter branch; and control means coupled to said selectively operable input header and to said first output line of said feed tank to selectively operate said input header to first deliver said solution to said first filter branch until said first filter branch is clogged and to operate said input header to deliver said solution to said second filter branch.

N. Apparatus as defined in claim 1, wherein said temperature correction means is a heat exchanger.

O. A method for the continuous hydrolyzation of lactose into glucose and galactose comprising the steps of: providing a continuous supply of a solution containing approximately 5% lactose in water; adjusting the pH of the solution to a predetermined value; pass said pH adjusted solution through a flow-through reactor having immobilized thereon and therein a lactase system whereby a substantial portion of said lactose is hydrolyzed into glucose and galactose.

P. A method as defined in claim O further comprising the step of heating the solution to a first predetermined temperature before introducing the solution to the flow through reactor to prevent the growth of bacteria in said solution.

Q. A method as defined in claim P further comprising the step of cooling the solution to a second predetermined temperature before introducing the solution to the flow-through reactor and after pasturization of said solution.

R. A method as defined in claim O, further comprising: the step of passing the solution through an ultra-violet radiation source before introducing the solution to the flow-through reactor to prevent the growth of bacteria in said solution.

S. A method as defined in claim R further comprising the step of adjusting the temperature of said solution before introducing the solution to the flow-through reactor.

T. A method as defined in claim O, further comprising: the step of filtering said solution before adjusting the pH of said solution to remove any particles that could block said flow-through reactor.

U. A method as defined in claim O, further comprising: the step of filtering said solution after adjusting the pH of said solution to remove any particles that would block said flow-through reactor.

V. A method as defined in claim O, further comprising the step of filtering said glucose, galactose and non-hydrolyzed solution before further use.

W. A method as defined in claims O or 10 or P or Q or R or S or T or U or V further comprising the step of passing a whey stream through an ultra-filtration device to remove the whey protein concentrate and employing the resulting whey stream as the lactose solution.

X. A method as defined in claims O or 10 or P or Q or R or S or T or U or V further comprising: the steps of passing a whey stream through a first ultra-filtration device to remove the whey protein concentrate and produce a first whey stream; and passing said first whey stream through a second ultra-filtration device to remove any residual protein and employing the resulting second whey stream as the lactose solution.

Y. A method as defined in claim X, comprising: the further step of passing said second whey stream through a turbidity meter and returning said second whey stream to said second ultra-filtration device if its turbidity is above a predetermined level.

CLAIMS

1.    Apparatus for the continuous hydrolyzation of lactose into glucose and galactose comprising: feed means for providing a continuous supply of a solution containing approximately 5% lactose in water; temperature control means coupled to said feed means to place said solution within a predetermined temperature range; pH control means coupled to said feed means to adjust the pH of said solution to a predetermined pH value; and flow through reactor means coupled to said temperature and pH control means to receive thereat said solution at said predetermined temperature and pH; said flow-through reactor means having a plurality of pores therein with a lactase system immobilized in and on said pores, said lactase system facilitating the hydrolyzation of a substantial portion of said lactose to glucose and galactose during the transit time of said solution through said flow-through reactor.

2.    Apparatus as defined in claim 1, further comprising: first filter means coupled between said feed means and said temperature and pH control means to remove any particles that could accumulate on said flow-through reactor means and impede its operation.

3.    Apparatus as defined in claim 1, further comprising: first filter means coupled between said temperature and pH control means and said flow-through reactor means to remove any particles that could accumulate on said flow-through reactor means and impede its operation.

4.    Apparatus as defined in claims 2 or 3 wherein said flow-through reactor is formed of a plurality of microporous sheets and said solution flows through said

- 29 -

0168127

reactor in a direction perpendicular to the major surfaces of said sheets; said first filter means removing substantially all of the particules in said solution to prevent said particules from blocking the pores of said microporous sheets and preventing the function thereof.

5. Apparatus as defined in Claim 1, wherein said flow-through reactor is formed of a plurality of microporous sheets and said solution flows through said reactor in a direction perpendicular to the major surfaces of said sheets.

6. Apparatus as defined in Claim 1, wherein said flow-through reactor means is in the form of a spiral jelly roll like form having a porous core and an open free end; first means coupling said reactor means porous core to said temperature and pH control means and second means coupled to said open free end of said reactor to conduct away from said reactor the glucose and galactose and any unconverted lactose solution.

7. Apparatus as defined in Claim 6, further comprising: first filter means coupled to said second means to remove all particles larger than a predetermined size.

8.    Apparatus as defined in claim 1, wherein said flow-through reactor means is in the form of a spiral jelly-roll like form having a porous core and an open free end; first means coupling said reactor means open free end to said temperature and pH control means and second means coupled to said porous core of said reactor to conduct away from said reactor the glucose and galactose and any unconverted lactose solution.

9.    Apparatus for the continuous hydrolyzation of lactose into glucose and galactose comprising: feed means for providing a continuous supply of a solution containing approximately 5% lactose in water; feed tank means having first, second and third input lines and first and second output lines, said first input line coupled to said feed means to permit the entry of said solution from said feed means; ultra-violet radiation means to irradiate said solution and prevent the growth of bacteria therein, said radiation means coupled between said second output and said second input lines to permit the solution in said feed tank means to be recirculated through said radiation means; pH control means coupled to said third input line to adjust the pH of said solution in said feed tank to a predetermined pH value; temperature control means coupled to said output line of said feed tank to place said solution within a predetermined temperature range; and flow through reactor means coupled to said temperature control means to receive thereat said solution at said predetermined temperature and pH; said flow-through reactor means having a plurality of pores therein with a lactose system immobilized in and on said pores, said lactase system facilitating the hydrolyzation of a substantial portion of said lactose to glucose and galactose during the transit time of said solution through said flow-through reactor.

10.    A method for the continuous hydrolyzation of lactose into glucose and galactose comprising the steps of: providing a continuous supply of a solution containing approximately 1 to 30% lactose in water; adjusting the pH of the solution to a predetermined value: pass said pH adjusted solution through a flow-through reactor having immobilized thereon and therein a lactase system whereby a substantial portion of said lactose is hydrolyzed into glucose and galactose.

FIG.1

FIG.2

FIG.7

1/3

0168127

FIG.3

FIG.4

FIG.5

0168127

FIG.6